# EUROPEAN PATENT SPECIFICATION

(11) **EP 0 521 951 B1**
(45) Date of publication and mention of the grant of the patent: **24.08.1994**
(21) Application number: 91906505.2
(22) Date of filing: 19.03.1991
(51) Int. Cl.: C12P 7/40

(54) **A PROCESS FOR DECOMPOSING PEROXYCARBOXYLIC ACIDS**
VERFAHREN ZUR ZERLEGUNG VON PEROXICARBONSÄUREN
PROCEDE PERMETTANT DE DECOMPOSER LES ACIDES PEROXYCARBOXYLIQUES

(30) Priority: 20.03.1990 DK 714/90
(43) Date of publication of application: 13.01.1993
(73) Proprietor: NOVO NORDISK A/S, 2880 Bagsvaerd (DK)
(72) Inventor: GODTFREDSEN, Sven, Erik, DK-3500 Vaerlose (DK); KIRK, Ole, DK-2200 Copenhagen N (DK); DAMHUS, Ture, DK-2100 Copenhagen (DK)
(74) Representative: Jorgensen, Dan
(86) International application number: DK9100084
(87) International publication number: WO9114783

(56) References cited:
- CHEMICAL ABSTRACTS, vol. 94, no. 25, 22 June 1981, Columbus, OH (US); T. ARAISO et al., p. 225, no. 204484m#
- CHEMICAL ABSTRACTS, vol. 93, no. 9, 01 September 1980, Columbus, OH (US); M.M. PALCIC et al., p. 269, no. 91006r#
- CHEMICAL ABSTRACTS, vol. 82, no. 17, 28 April 1975, Columbus, OH (US); P. JONES et al., p. 185, no. 108080h#

## Description

### FIELD OF INVENTION

The present invention relates to a process for decomposing peroxycarboxylic acids by treatment with a catalyst.

### BACKGROUND OF THE INVENTION

Peroxycarboxylic acids constitute an important class of substances which may be used for a wide range of purposes. Thus, peroxycarboxylic acids are commonly employed as oxidizing reagents in the field of organic synthesis for the production of a variety of organic chemicals. In particular, organic molecules containing unsaturations or other reducing groups may be oxidized in a high yield under mild conditions when subjected to peroxycarboxylic acids during organic synthesis, and consequently peroxycarboxylic acids may advantageously be used, e.g., for the preparation of epoxides from unsaturated hydrocarbons. Apart from their use as oxidation reagents, peroxycarboxylic acids may also be used as bleaching agents, for instance to decolourize paper mill process streams. Furthermore, preparations of peroxycarboxylic acids may be used for disinfection purposes (e.g. in the food industry) due to the sensitivity of microorganisms to these compounds (see e.g. H.C. Flemming, Zbl.Bakt.Hyg., I.Abt.Orig. B 179, 97-111 (1984)).

While peroxycarboxylic acids are highly advantageous reagents for these and other applications, their continued presence in the end products is not always desirable as they represent a potential environmental or health hazard. For instance, solutions of dyes decolourized by means of peroxycarboxylic acids should preferably be free from remaining peroxycarboxylic acid before being discharged into the environment where the peroxycarboxylic acid might exert a harmful effect. Similarly, utensils and surfaces disinfected in the food industry by means of peroxycarboxylic acids should be free from the reagent prior to use since the peroxycarboxylic acid may otherwise deteriorate the food product produced or eventually reach the consumer. Furthermore, substances prepared by chemical synthesis utilizing peroxycarboxylic acids should be free from the oxidation reagent which for instance might undesirably influence the further use or conversion of the synthesized substance.

Accordingly, there is a need for processes whereby residual peroxycarboxylic acids may be removed, in particular processes which allow for selective decomposition of peroxycarboxylic acids under mild conditions and which may thus be applied for the removal of peroxycarboxylic acids without adversely affecting other components in the peroxycarboxylic acid containing medium. Such processes should preferably also be economically viable for the treatment of large volumes of very dilute peroxycarboxylic acid containing solutions. To the present inventors' knowledge, no entirely satisfactory processes for the removal of peroxycarboxylic acids are currently available, and the use of peroxycarboxylic acids is accordingly limited, no matter how desirable their application in many processes may be.

### SUMMARY OF THE INVENTION

It has surprisingly been found that certain enzymes may advantageously be used for the conversion of peroxycarboxylic acids to the corresponding carboxylic acids. Such enzymatic processes permit selective transformation of peroxycarboxylic acids under mild conditions and provide an economical way of removing peroxycarboxylic acids from large volumes of dilute solutions of the peroxycarboxylic acids.

Accordingly, the present invention relates to a process for converting peroxycarboxylic acids to carboxylic acids, the process comprising treating a peroxycarboxylic acid of the general formula I:

R-CO-OOH (I)

wherein R is a linear or branched alkyl group, an aryl group or an aryl-alkyl group each of which may optionally be substituted with one or more hydroxy, halogen, alkoxy, amino, alkylamino, sulfoxy, sulfono, amido, carboxy, percarboxy or nitro groups, with an enzyme catalyst to form the corresponding carboxylic acid of the general formula II:

R-CO-OH (II)

The use of enzymes (especially hydrolases) has previously been proposed for the formation of peroxycarboxylic acids in aqueous solutions. Thus German Patent 2 240 605 (Colgate-Palmolive Co.) suggests the use of hydrolases, in particular esterases or lipases of plant or animal origin, for generating peroxycarboxylic acids from simple ester substrates. Likewise the use of lipases in general and a lipase derived from Pseudomonas putida in particular for generating peroxycarboxylic acids from ester substrates is described in EP 253 487 and EP 268 456 (both to Clorox). A similar use of proteases is described in EP 310 952 (Henkel). To the best of the present inventors' knowledge, however, it has not previously been proposed to degrade peroxycarboxylic acids by means of such enzymes.

### DETAILED DESCRIPTION OF THE INVENTION

Enzymes which may be employed as catalysts in the process of the invention are preferably hydrolases such as proteases, esterases and lipases. From the point of view of cost and availability it is advantageous to employ enzymes producible by microorganisms such as yeasts, bacteria or fungi.

Lipases which may be employed in the present process may be microbial lipases produced, for instance, by strains of Aspergillus, Enterobacterium, Chromobacterium, Geotricium or Penicillium. Preferred lipases for use according to the invention are those produced by species of Aspergillus, Mucor, Humicola, Pseudomonas or Candida.

Particularly preferred lipases are those produced by the following strains of microorganisms:
Humicola lanuginosa, DSM 3819 and 4109,
Humicola brevispora, DSM 4110,
Humicola brevis var. thermoidea, DSM 4111, and
Humicola insolens, DSM 1800.

To produce the lipase, the strain producing the enzyme may be cultivated under aerobic conditions in a nutrient medium containing assimilable carbon and nitrogen sources as well as essential minerals, trace elements etc., the medium being composed according to established practice. After cultivation, liquid enzyme concentrates may be prepared by removing insoluble materials, e.g. by filtration or centrifugation, after which the culture broth may be concentrated by evaporation or reverse osmosis. Solid enzyme preparations may be prepared from the concentrate by precipitation with salts or water-miscible solvents, e.g. ethanol, or by drying such as spray-drying in accordance with well-known methods.

Other lipases which may be used in the process of the invention are commercial lipases, e.g., those known under the trade names Palatase® (a lipase obtainable from Aspergillus niger, available from Novo Nordisk A/S) and Lipolase™ (also obtainable from Novo Nordisk A/S).

The lipase may furthermore be one producible by recombinant DNA techniques, cf. for instance EP 238 023.

The esterase employed in the process of the invention may be of animal or microbial origin. A suitable esterase for the present purpose is one obtainable from hog liver.

When employed in the process of the invention, the enzyme may be in a soluble state. It is, however, preferred to immobilize the enzyme on a solid support in order to facilitate the separation of the enzyme from the material treated with peroxycarboxylic acid. Immobilization procedures are well known (cf. for instance K. Mosbach, ed., "Immobilized Enzymes", Methods in Enzymology 44, Academic Press, New York, 1976) and include cross-linking of cell homogenates, covalent coupling to insoluble organic or inorganic supports, entrapment in gels and adsorption to ion exchange resins or other adsorbent materials. Coating on a particulate support may also be employed (cf. for instance A.R. Macrae and R.C. Hammond, Biotechnology and Genetic Engineering Reviews 3, 1985, p. 193). Suitable support materials for the immobilized enzyme are, for instance, plastics (e.g. polypropylene, polystyrene, polyvinylchloride, polyurethane, latex, nylon, polytetrafluoroethylene, polyethylene terephthalates, polyvinylacetate, polyvinylalcohol or any suitable copolymer thereof), polysaccharides (e.g. agarose or dextran), ion exchange resins (both cation and anion exchange resins), silicon polymers (e.g. siloxane) or silicates (e.g. glass).

It is preferred to immobilize the enzyme on an ion exchange resin by adsorbing the enzyme to the resin or by cross-linking it to the resin by means of glutaraldehyde or another cross-linking agent in a manner known per se. A particularly preferred resin is a weakly basic anion exchange resin which may be a polystyrene-, acrylic- or phenol-formaldehyde-type resin. Examples of commercially available resins are Lewatit E 1999/85 or E2001/85 (registered trademark of Bayer, Federal Republic of Germany) and Duolite ES-568 (registered trademark of Rohm & Haas, FRG). Immobilization of enzymes to acrylic-type resins may be carried out according to EP 140 542. Immobilization to phenol-formaldehyde-type resins may be done according to DK 85/878.

Another convenient material for immobilizing enzymes is an inorganic support, such as a silicate. The enzyme may be attached to the support by adsorption or by covalent coupling, e.g. as described in K. Mosbach, ed., op. cit.

R in the general formulae I and II above is preferably a linear alkyl group, in particular a linear alkyl group with 1-19 carbon atoms, preferably 1-13 carbon atoms, more preferably 1-10 carbon atoms and most preferably 2 or 8-10 carbon atoms. R may also be an alkyl group substituted by one or more halogen atoms, preferably chloro atoms.

According to the invention, the conversion of the peroxycarboxylic acid (I) to the carboxylic acid (II) may be carried out in an aqueous medium. This is an advantage when the peroxycarboxylic acid treatment preceding the present process takes place in, for instance, an aqueous solution, such as a solution of pigments or a solution of peroxycarboxylic acid used for disinfection purposes.

The process of the invention is particularly useful in connection with bleaching processes, disinfection processes and organic synthesis processes involving the use of peroxycarboxylic acids. In these instances any peroxycarboxylic acid remaining after the oxidation process may conveniently be removed by treatment with the enzyme catalyst. Since enzymes exhibit a high affinity for their substrates (in this case the peroxycarboxylic acids) and a high degree of specificity, the conversion of the peroxycarboxylic acid may be carried out substantially without affecting any other compounds present, and may furthermore be used to remove even small amounts of peroxycarboxylic acid.

Accordingly, the process of the present invention may suitably be employed for the conversion of the peroxycarboxylic acid (I) or residues thereof to the carboxylic acid (II) in a step subsequent to disinfecting an object (e.g. a utensil or a surface) with the peroxycarboxylic acid (I). In another favoured embodiment, the process of the invention may be employed for the conversion of the peroxycarboxylic acid (I) or residues thereof to the carboxylic acid (II) in a subsequent step to bleaching a coloured substance with the peroxycarboxylic acid (I). In yet another favoured embodiment, the process of the invention may be employed for the conversion of the peroxycarboxylic acid (I) or residues thereof to the carboxylic acid (II) in a subsequent step to the oxidation of organic or inorganic substrates with a peroxycarboxylic acid (I).

Certain enzymes, when employed according to the invention, will give rise to the formation of hydrogen peroxide concomitantly with conversion of the peroxycarboxylic acid as shown in Reaction Scheme 1 below in which R has the meaning indicated above: 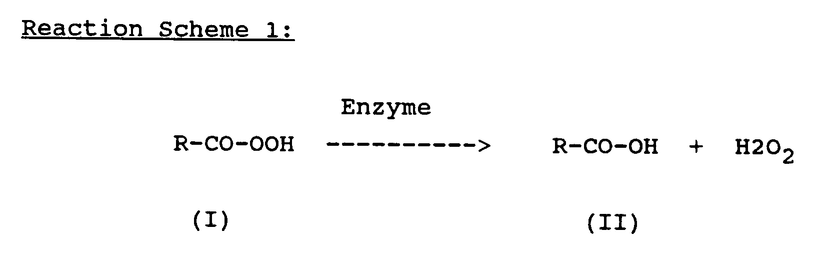
In some cases, it may be advantageous to convert the hydrogen peroxide formed in the reaction with an enzyme with a view to removing this oxidizing species from the reaction mixture or initiating oxidizing processes in the reaction medium, based on the use of hydrogen peroxide. Accordingly, it may be advantageous to carry out the conversion of the peroxycarboxylic acid (I) in the presence of a hydrogen peroxide converting enzyme or, alternatively, to subject the hydrogen peroxide formed as a result of the conversion of the peroxycarboxylic acid (I) to subsequent treatment with a hydrogen peroxide converting enzyme. Examples of suitable hydrogen peroxide converting enzymes are catalase, peroxidases, and haloperoxidases.

The process of the invention is further illustrated by the following examples which are not intended to be in any way limiting to the scope of the invention as claimed.

### EXAMPLES

### General Methods

Peroxycarboxylic acids were prepared according to the method described by W. E. Parker, C. Ricciuti, C. L. Ogg and D. Swern, **J. Am. Chem. Soc.** 77, 4037 (1955) except for peracetic acid which was obtained as an approximately 2 M solution under the trademark Proxitane 507. Peroxycarboxylic acid levels may be determined, also when hydrogen peroxide is present together with the peroxycarboxylic acid, by methods known in the art, e.g. by iodometry at 5^{o}C as described by Sully and Williams in Analyst, 1962, **87**, 653 (this method also gives the concentration of hydrogen peroxide). One Lipase Unit (LU) is the amount of enzyme which liberates one micromole of butyric acid per minute from tributyrin as a substrate in a pH-stat at 30^{o}C and pH 7 (a detailed description of the assay (AF 95) is available from Novo Nordisk A/S upon request).

### Example 1.

To a 0.47 mM solution of peroctanoic acid in a 50 mM phosphate buffer at pH 7 and 40^{o}C was added 10 LU/ml Palatase^{R} (a fungal lipase produced by fermentation of a strain of Aspergillus niger), and the peroxycarboxylic acid concentration was monitored as a function of time (the numbers in brackets refer to a reference-experiment performed under similar conditions but without addition of enzyme):

| Time | Peracid concentration (mM) | |
|---|---|---|
| Before addition of enzyme | 0.47 | (0.50) |
| 3 min | 0*) | (0.50) |
| 9 min | 0*) | (0.48) |

| | | |
|---|---|---|
| *) Not detectable. | | |

### Example 2

To a 0.56 mM solution of peracetic acid in a 50 mM phosphate buffer at pH 7 and 40^{o}C was added 10 LU/ml Palatase^{R} (as described in example 1) and the peroxycarboxylic acid concentration was monitored as a function of time :

| Time after addition of enzyme (min) | Peracid concentration (mM) |
|---|---|
| 0 | 0.56 |
| 4 | 0.26 |
| 9 | 0.08 |
| 14 | 0 (not detectable) |

In a similar experiment, but without any addition of enzyme, the peroxycarboxylic acid concentration declined much more slowly as shown in the following table :

| Time (min) | Peracid concentration (mM) |
|---|---|
| 0 | 0.61 |
| 5 | 0.57 |
| 10 | 0.55 |
| 15 | 0.53 |

### Example 3

To a 0.2 mM solution of peracetic acid in a 50 mM phosphate buffer pH 8 and 25^{o}C was added 50 U/ml (Sigma units) Esterase from Hog Liver (Sigma E-3128, 6365 U/ml) and the peroxycarboxylic acid concentration was monitored as a function of time :

| Time after addition of enzyme (min) | Peracid concentration (mM) |
|---|---|
| 0 | 0.20 |
| 2 | 0.15 |
| 10 | 0 (not detectable) |
| 18 | 0 (not detectable) |

In a corresponding experiment with no enzyme, the peracetic acid concentration did not change within 18 min.

### Example 4

To a solution of 0.5 mM pernonanoic acid in 15 mM phosphate buffer at pH 8.5 and 22^{o}C was added 5 LU/ml Lipolase™ (available from Novo Nordisk A/S) and both the peroxycarboxylic acid concentration and the concentration of H₂O₂ was monitored as a function of time:

| Time | Concentrations (mM) | |
|---|---|---|
| | Pernonanoic acid | H₂O₂ |
| Immediately after addition of enzyme | 0.30 | 0.08 |
| 13 min later | 0 (not detectable) | 0.32 |

In a corresponding experiment with no enzyme, the peroxycarboxylic acid concentration was unchanged after 14 min.

## Claims

1. A process for converting peroxycarboxylic acids to carboxylic acids in an aqueous medium, the process comprising treating a peroxycarboxylic acid of the general formula I:
R-CO-OOH (I)
wherein R is a linear or branched alkyl group, an aryl group or an aryl-alkyl group each of which may optionally be substituted with one or more hydroxy, halogen, alkoxy, amino, alkylamino, sulfo, sulfoxy, sulfono, amido, carboxy, percarboxy or nitro groups, with an enzyme which is a hydrolase to form the corresponding carboxylic acid of the general formula II:
R-CO-OH (II)
wherein R has the meaning indicated above.

2. A process according to claim 1, wherein the enzyme is an esterase, a protease or a lipase.

3. A process according to claim 2, wherein the enzyme is a microbial lipase.

4. A process according to claim 3, wherein the lipase is one obtainable from a strain of Mucor, Aspergillus, Humicola, Pseudomonas or Candida.

5. A process according to any of claims 1-4, wherein the enzyme is immobilized on a solid support.

6. A process according to any of claims 1-5, wherein R is a linear alkyl group, in particular a linear alkyl group with 1-19 carbon atoms, preferably with 1-13 carbon atoms, more preferably with 1-10 carbon atoms and most preferably with 2 or 8-10 carbon atoms.

7. A process according to any of claims 1-6, wherein R is an alkyl group substituted with one or more halogen atoms, preferably chlorine atoms.

8. A process according to any of claims 1-7, wherein the conversion of the peroxycarboxylic acid (I) or residues thereof to the carboxylic acid (II) is a subsequent step to disinfecting an object with the peroxycarboxylic acid (I).

9. A process according to any of claims 1-7, wherein the conversion of the peroxycarboxylic acid (I) or residues thereof to the carboxylic acid (II) is a subsequent step to bleaching a coloured substance with the peroxycarboxylic acid (I).

10. A process according to any of claims 1-7, wherein the conversion of the peroxycarboxylic acid (I) or residues thereof to the carboxylic acid (II) is a subsequent step to the oxidation of organic or inorganic substrates with a peroxycarboxylic acid (I).

11. A process according to any of the preceding claims, wherein the conversion of the peroxycarboxylic acid (I) is carried out in the presence of or followed by treatment with a hydrogen peroxide decomposing enzyme.

12. A process according to claim 11, wherein the hydrogen peroxide decomposing enzyme is a catalase, a peroxidase or a haloperoxidase.

## Patentansprüche

1. Ein Verfahren zur Umwandlung von Peroxycarbonsäuren zu Carbonsäuren in einem wäßrigen Medium, wobei das Verfahren umfaßt, daß eine Peroxycarbonsäure der allgemeinen Formel I:
R-CO-OOH (I),
in der R eine lineare oder verzweigte Alkylgruppe, eine Arylgruppe oder eine Arylalkylgruppe ist, von denen jede fakultativ mit einer oder mehreren Hydroxy-, Halogen-, Alkoxy-, Amino-, Alkylamino-, Sulfo-, Sulfoxy-, Sulfono-, Amido-, Carboxy-, Percarboxy- oder Nitrogruppen substituiert sein kann, mit einem Enzym behandelt wird, das eine Hydrolase ist, um die entsprechende Carbonsäure der allgemeinen Formel II zu bilden:
R-CO-OH (II),
in der R die oben angegebene Bedeutung hat.

2. Ein Verfahren nach Anspruch 1, wobei das Enzym eine Esterase, eine Protease oder eine Lipase ist.

3. Ein Verfahren nach Anspruch 2, wobei das Enzym eine mikrobielle Lipase ist.

4. Ein verfahren nach Anspruch 3, wobei die Lipase eine ist, die aus einem Stamm von Mucor, Aspergillus, Humicola, Pseudomonas oder Candida gewonnen werden kann.

5. Ein Verfahren nach einem der Ansprüche 1 - 4, wobei das Enzym auf einem festen Träger immobilisiert ist.

6. Ein Verfahren nach einem der Ansprüche 1 - 5, wobei R eine lineare Alkylgruppe ist, insbesondere eine lineare Alkylgruppe mit 1-19 Kohlenstoffatomen, vorzugsweise mit 1-13 Kohlenstoffatomen, bevorzugter mit 1-10 Kohlenstoffatomen und am bevorzugtesten mit 2 oder 8-10 Kohlenstoffatomen.

7. Ein Verfahren nach einem der Ansprüche 1-6, wobei R eine Alkylgruppe ist, die mit einem oder mehreren Halogenatomen, vorzugsweise Chloratomen, substituiert ist.

8. Ein Verfahren nach einem der Ansprüche 1-7, wobei die Umwandlung der Peroxycarbonsäure (I) oder von Rückständen derselben zu der Carbonsäure (II) ein anschließender Schritt an die Desinfektion eines Gegenstandes mit der Peroxycarbonsäure (I) ist.

9. Ein Verfahren nach einem der Ansprüche 1-7, wobei die Umwandlung der Peroxycarbonsäure (I) oder von Rückständen derselben zu der Carbonsäure (II) ein anschließender Schritt an das Bleichen einer gefärbten Substanz mit der Peroxycarbonsäure (I) ist.

10. Ein Verfahren nach einem der Ansprüche 1-7, wobei die Umwandlung der Peroxycarbonsäure (I) oder von Rückständen derselben zu der Carbonsäure (II) ein anschließender Schritt an die Oxidation von organischen oder anorganischen Substraten mit einer Peroxycarbonsäure (I) ist.

11. Ein Verfahren nach einem der vorangehenden Ansprüche, wobei die Umwandlung der Peroxycarbonsäure (I) in der Gegenwart von oder gefolgt von der Behandlung mit einem Wasserstoffperoxid zersetzenden Enzym durchgeführt wird.

12. Ein Verfahren nach Anspruch 11, wobei das Wasserstoffperoxid zersetzende Enzym eine Katalase, eine Peroxidase oder eine Haloperoxidase ist.

## Revendications

1. Procédé pour la conversion d'acides peroxycarboxylique en acides carboxyliques dans un milieu aqueux, le procédé comprenant le traitement d'un acide peroxycarboxylique de formule générale I :
R-CO-OOH (I)
dans laquelle R est un groupe alkyle linéaire ou ramifié, un groupe aryle ou un groupe arylalkyle dont chacun peut être facultativement substitué par un ou plusieurs groupes hydroxy, halogène, alcoxy, amino, alkylamino, sulfo, sulfoxy, sulfono, amido, carboxy, percarboxy ou nitro, avec une enzyme qui est une hydrolase pour former l'acide carboxylique correspondant de formule générale II :
R-CO-OH (II)
dans laquelle R a la signification désignée ci-dessus.

2. Procédé selon la revendication 1, dans lequel l'enzyme est une estérase, une protéase ou une lipase.

3. Procédé selon la revendication 2, dans lequel l'enzyme est une lipase microbienne.

4. Procédé selon la revendication 3, dans lequel la lipase est une enzyme pouvant être obtenue à partir d'une souche de Mucor, Aspergillus, Humicola, Pseudomonas ou Candida.

5. Procédé selon l'une quelconque des revendications 1-4, dans lequel l'enzyme est immobilisée sur un support solide.

6. Procédé selon l'une quelconque des revendications 1-5, dans lequel R est un groupe alkyle linéaire, en particulier un groupe alkyle linéaire avec 1-19 atomes de carbone, de préférence avec 1-13 atomes de carbone, de façon plus préférée avec 1-10 atomes de carbone et de façon la plus préférée avec 2 ou 8-10 atomes de carbone.

7. Procédé selon l'une quelconque des revendications 1-6, dans lequel R est un groupe alkyle substitué par un ou plusieurs atomes halogène, de préférence des atomes de chlore.

8. Procédé selon l'une quelconque des revendications 1-7, dans lequel la conversion de l'acide peroxycarboxylique (I) ou de ses résidus en l'acide carboxylique (II) est une étape consécutive à la désinfection d'un objet avec l'acide peroxycarboxylique (I).

9. Procédé selon l'une quelconque des revendications 1-7, dans lequel la conversion de l'acide peroxycarboxylique (I) ou de ses résidus en l'acide carboxylique (II) est une étape consécutive au blanchiment d'une substance colorée avec l'acide peroxycarboxylique (I).

10. Procédé selon l'une quelconque des revendications 1-7, dans lequel la conversion de l'acide peroxycarboxylique (I) ou de ses résidus en l'acide carboxylique (II) est une étape consécutive à l'oxydation de substrats organiques ou inorganiques avec un acide peroxycarboxylique (I).

11. Procédé selon l'une quelconque des revendications précédentes, dans lequel la conversion de l'acide peroxycarboxylique (I) est conduite en présence de ou suivie du traitement d'une enzyme décomposant le peroxyde d'hydrogène.

12. Procédé selon la revendication 11, dans lequel l'enzyme décomposant le peroxyde d'hydrogène est une catalase, une peroxydase ou une haloperoxydase.
